# EUROPEAN PATENT APPLICATION

(11) **EP 2 865 331 A1**
(43) Date of publication of application: **29.04.2015**
(21) Application number: 13189705.0
(22) Date of filing: 22.10.2013
(51) Int. Cl.: A61B 5/145, A61B 5/00, A61B 10/02, A61B 19/00, G01N 1/00, G01N 21/64

(54) **Method for providing information on a relative position of a tissue sample**

(71) Applicant: Siemens Aktiengesellschaft, 80333 München (DE)
(72) Inventor: Ladic, Lance Anthony, Robinsville, 08691 (US); Rausch, Saskia, Dr., 10715 Berlin (DE)

(57) **Abstract**

The invention relates to a method for providing information on a relative position of a tissue sample (14), wherein the tissue sample (14) is a collected part of a tissue of interest (10) of a patient (12), comprising the steps of providing an image (22) of the labeled tissue (10) of interest using a sensor device (20, S2), determining a collection site (18) on the tissue of interest (10) by using the provided image (22), and, according to the determined excision site (18), determining a relative position of the tissue sample (14). A labeling of the tissue (10) may be performed using e.g. a rinsing agent or a biopsy device as labeling device (16).

## Description

The invention relates to a method for providing information on a relative position of a tissue sample in, e.g., a telemedical approach, wherein the tissue sample is an collected part of a tissue of interestwithin a patient.

Oral cancer is one of the most common cancers worldwide and the one with the highest mortality rate of malignancies. In developing countries, oral cancer is a significant problem due to the high use of tobacco or chewing of e.g. betel nuts or other substances that have carcinogenic ingredients. Oral cancer can be detected by brush biopsy, a minimally-invasive biopsy method, combined with microscopic analysis. Brush biopsy is a fast and painless method for oral tissue sampling. The gathered cells can be used for diagnostics of cellular changes e.g. premalignant and malignant (cancerous) processes. For diagnostics, normally light microscopy is used to interpret related samples. Normally, a lesion in a patient's mouth is the first sign for a possible oral cancer. A biopsy is therefore taken from the area of the lesion and analyzed to check for presence of disease.

Diagnosis of oral cancer normally requires a well-trained medical professional with microscopy expertise in a clinic or dentist's office. The medical professional first inspects the patient's mouth in detail. If he/she discovers a lesion, a biopsy (either a traditional and invasive "punch" or brush method) will be performed. For the diagnostics and analysis of cellular material gathered by biopsy, light microscopy is typically used. A reliable diagnosis is therefore only performed in a well-equipped laboratory by well-trained medical personnel.

A tool for use in a telemedical approach, especially for people in rural areas where no or limited trained medical personnel is present, has been developed by the Stanford University. A scanning device for a smartphone can be used to take panoramic images of the oral cavity. If malignant cancer lesions are illuminated by light that is emitted by the device, they appear as regions with differential contrast. An image taken by the device can be sent wirelessly to e.g. a dentist for diagnosis.

Yet, the patient or the medical personnel in the rural region may not always know how to best examine a patient's mouth and decide where to perform a biopsy. Limited trained personnel may also have difficulties in evaluating the quality of such biopsy, which may result in false-negative diagnosis results.

It is an object underlying the present invention to provide a method to control the diagnosis and/or biopsy process.

This object is reached by the methods, and the labeling device according to the independent patent claims. Advantageous further embodiments are given by the dependent claims.

The method according to the invention for providing information on a relative position of a tissue sample, wherein the tissue sample is a collected part of a tissue of interest of a human or animal patient, comprising the steps of:
- providing an image, e.g. a digital photograph or an image of a video sequence, of the tissue of interest using a sensor device,
- determining a collection site on the tissue of interest by using the provided image, and
- according to the determined excision site, determining the relative position of the tissue sample. Thus, monitoring of the biopsy and/or determining whether the biopsy was duly executed can take place. Also, it can be ensured that e.g. a field technician took the biopsy from an appropriate location of the tissue of interest, e.g. by confirming this with a remote clinician.

A sensor device is a device that measures a physical quantity, preferably light, and converts it to a signal. A preferred sensor device according to the invention comprises a scanner, a photo camera or frame grabber or a video camera, i.e. a device that can capture a digital image or series of images. The step of providing the image of the tissue of interest is useful for showing the visible information on the image to e.g. a person that is located in a remote location or for saving the information so that the patient does not need to be present at the place or time of determining the collection site. Determining the relative position of the tissue sample enables the correlation of, e.g. a microscope image of the tissue sample with the macroscopic anatomy of an organ that comprises the tissue sample.

In addition to the use of e.g. brush biopsy and microscopic analysis, a macroscopic view into the patient's mouth that reveals the position of a suspect oral lesion with respect to the area sampled by e.g. brush biopsy is very important. In addition to facilitating monitoring a biopsy of a tissue sample, the method for providing information on a relative position of a tissue sample also comprises correlating microscopic observations of a biopsy (or biopsies) with the macroscopic location of where the biopsy (or biopsies) was (were) acquired. Doing this for telemedicine approaches (i.e. from a remote location) is one use case, but it may also be performed locally.

The invention provides a method for monitoring the biopsy and/or determining whether the biopsy whether the biopsy was properly performed. The method according to the invention also improves the reliability of diagnosis or other patient triaging, and provides a preventative screening of a patient's tissue. Thereby, the method of the invention does not necessarily involve a diagnosis as such.

The invention helps to improve the acquisition of e.g. a brush biopsy sample taken by less experienced personnel, e.g. in a nonclinical environment, or remote location, or the patient by providing a telemedicine framework that allows them send images of the region to remote experts for interpretation and support. Of course, as an alternative, the method of the invention may also be performed locally, e.g. in a doctor's or dentist's office or operating room, i.e. to have a record of associating where the biopsy or biopsies came from in relation to the source tissue.

In a further preferred embodiment of the invention, the tissue of interest comprises the collection site and determining the collection site comprises determining the site where the tissue sample has been collected so that e.g. correlating a microscopic image of the tissue sample with the macroscopic anatomy can take place even when the patient is spatially or temporally absent. Alternatively, the tissue of interest is unaffected and determining the collection site comprises determining the site where the tissue sample is to be collected, e.g. excised. Thus, the acquisition process of the biopsy sample, e.g. when performed by untrained medical personnel, or the patient, can be supervised and controlled. Preferably, the image is part of an image sequence, preferably a video image sequence.

Preferably, according to a further embodiment of the invention, the method comprises the step of labeling the tissue of interest prior to providing the image of the labeled tissue of interest using a labeling means. In this way, the contrast between different structures within the tissue is enhanced (e.g. between a tumor (or a lesion) and normal tissue) and a special tissue region can be accentuated.

The tissue of interest may, according to a further embodiment of the invention, be labeled by irradiating the tissue of interest with fluorescent light, and/or irradiating or illuminating the tissue of interest with light, and capturing a fluorescence emitted by the tissue using a fluorescence sensor. In this case, the labeling device may, for example, comprise a scanning device comprising a light source, in particular a scanning device as described above. Only sites of the tissue with e.g. a specific cell structure or a specific cell type are thus labeled. Using a scanning device as described above provides a panoramic image of the tissue in question.

The reliability of the labeling of the tissue of interest, e.g. a tissue in the oral captivity, wherein said scanning device is used, depends on a successful operation of said scanning device. In order to avoid the dependency on a device of high technical standard, the labeling can, according to a preferred embodiment of the invention, be performed by labeling the tissue with a rinsing agent comprising a stain or dye before the tissue sample is collected so that the labeling process can be performed quickly and cheaply. Alternatively, the tissue of interest is labeled with a biopsy device comprising a stain or dye, e.g. a dye- or other label-impregnated or -coated brush biopsy wand, as labeling device. This allows for a quick and reliable labeling process. Also, no additional biopsy tool is necessary. These are preferred embodiments of the method of the invention, as in both cases no complex technology is needed.

In a further embodiment of the invention, the method comprises the step of providing the tissue sample and/or an image of the tissue sample. Determining the relative position of the tissue sample then involves using the provided image of the tissue sample and/or the provided tissue sample so that the interpretation of the tissue sample is improved and may be used for a telemedical approach. An optional step of determining a trait of a cell type of the tissue sample according to a further embodiment of the invention supports such interpretation of the tissue sample.

In a further embodiment, the tissue sample may be evaluated. Therefore, the tissue sample may be spread onto a glass slide, and be put under a microscope for evaluation. An image of the slide and/or the tissue sample may be taken with the microscope, e.g. a conventional microscope, or a variant thereof, such as e.g. a smartphone microscope, that has the capability of capturing e.g. digital images, e.g. with a digital camera connectedto the microscope. The image or the images may be evaluated by a clinican, and/or by computer-aided image analysis.

According to a further embodiment of the invention, at least one step of providing an image includes transmitting the image via a wireless or wire-bound communication connection, i.e. data connection, e.g. via W-Lan or a cable, to a control device, in particular to a remote control device. A control device is an electronic device for receiving images, in particular digital images or digital image sequences, such as a computer or a microprocessor. This step serves to e.g. process the image, and also supports an online interpretation by a telemedical approach.

The object mentioned above is further solved by a method for diagnosing a pathological condition of a tissue sample, comprising the steps of labeling a tissue of interest with fluorescent light, a stain or dye using a labeling device, providing an image of the labeled tissue of interest, determining a collection site on the labeled tissue of interest by using the provided image, and determining a pathological condition of the ex situ tissue sample subject to the determined excision site. A further embodiment of such method for diagnosing a pathological condition of a tissue sample comprises the step of obtaining the tissue sample from the tissue of interest of a patient is performed or after labeling the tissue of interest. Obtaining the tissue sample may e.g. comprise a punch biopsy or a brush biopsy, preferably a brush biopsy. Further embodiments may include single method steps as already described above.

The object is also solved by a labeling means for use in any of the previously described methods. Such labeling device comprises a stain or dye, and a biopsy brush for applying the tissue stain or dye on the tissue of interest. Alternatively, or additionally, the labeling device comprises a rinsing agent, and a stain or dye.

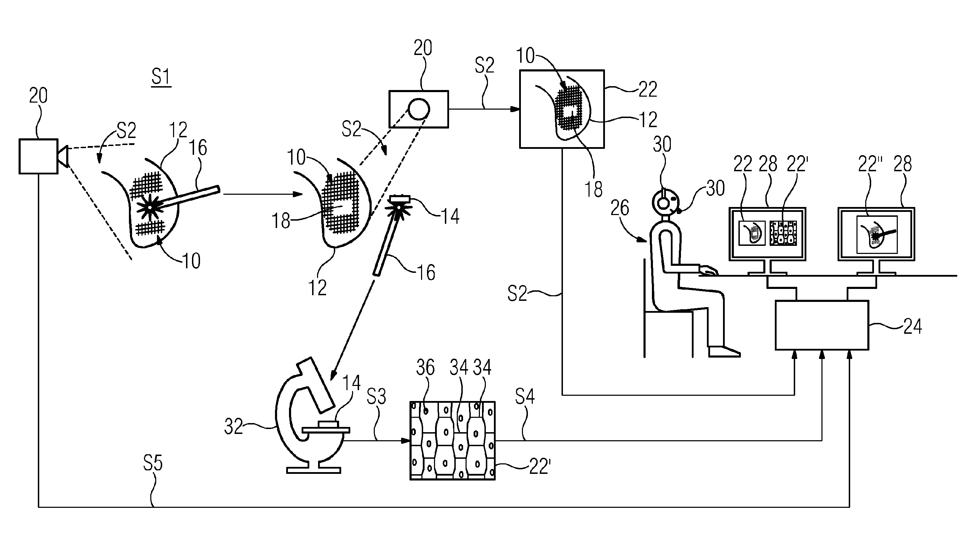

Below, the invention and its embodiments are explained in detail with reference to the drawings. The below examples describe preferred embodiments of the invention. Analog elements exhibit the same reference signs. The single drawing shows
a sketch of the principle of a method of the invention according to one embodiment.

The drawing shows a tissue of interest 10, for example the surface of a tongue or an oral mucosa of a patient 12. As an example, patient 12 lives in a remote region where medical facilities are rare and not well equipped, but where a medical assistant or field technician offers to examine patients with the help of telemedical assistance. Before setting up an individual therapy for patient 12, the medical assistant who has the suspicion that patient 12 may be at risk for oral cancer, seeks for a confirmation of his diagnosis from a remote clinic and/or likes to have the reliability of a possible biopsy checked. Such biopsy would comprise excising a tissue sample 14 using a biopsy device, e.g. a biopsy punch, or a biopsy brush.

In an optional procedural step S1, the tissue of interest 10 is labeled using a labeling device as labeling means 16. The labeled tissue of interest 10 is shown as shaded area in the drawing. As punching a tissue sample 14 may be very painful, use of a minimally-invasive biopsy brush as shown in the drawing is a preferred tool for biopsy, and thus a preferred labeling means 16. The exemplary labeling device 16 of the drawing is a biopsy brush that is impregnated with the stain or dye, which remains on the tissue after the brush was used. This facilitates a quick and reliable labeling process. The stain or dye may e.g. comprise a color marker or fluorescent marker, such as e.g. Alcian blue stain or Toluidine blue stain for staining e.g. epithelial tissue cells or a specific cell type such as e.g. cancer cells. Preferably, the labeling device 16 is disposable. Such labeling means 16 may also be used for collecting the tissue sample 14, thus enabling a person 12 without medical training to perform both labeling, and obtaining the tissue sample 14.

Alternatively, the labeling means 16 may comprise a rinsing agent, in particular a mouth rinse that comprises a stain or dye. A mouth rinse that is used to stain plaques is known in the art and may, as an example, be used in the method of the invention. Other mouth rinses and stains as already known by the skilled person may alternatively be used. Such mouth rinse is preferably applied before the biopsy.

By collecting the tissue sample 14, the labeled part of tissue 10 is removed, and thus a collection site 18 is unlabeled. The method for providing information on a relative position of a tissue sample according to the invention disclosed herein does, however, not comprise the excision step itself.

A sensor device 20, e.g. a digital photo camera, provides an image 22 of the tissue of interest 10 (S2). Providing the image (S2) may comprise the steps of capturing the image 22 and/or transferring the image 22 to e.g. a control device 24 using e.g. a data connection, e.g. a cable or a wireless data connection, or a communication network using common communication standards.

A captured image 22, 22', 22", if not natively in digital format, may be converted into digital format for storage and/or transmission to be used for further assessment. As an example, a smartphone can be used to take an image 22 as a macroscopic picture e.g. of the oral cavity, and/or - if connected to a microscope 32 - a microscopic image 22' of the tissue sample 14. As an example, a panoramic image of e.g. the oral cavity may be captured before a biopsy, or biopsies, are taken. In order to generate a e.g. a smartphone microscope image 22', the tissue sample 14 is e.g. first put onto a slide, then the slide is put onto a microscope stage and then captured. This step can also be done away from the patient 12, i.e. the patient 12 does not necessarily have to be present. The sensor device may also e.g. be a sensor device of a mobile device, e.g. of a smartphone. The camera can then capture a digital image 22' of the tissue sample 14.

The control device 24 preferably comprises a remote computer or microprocessor. In the example of the drawing, the control device 24 comprises a remote computer of e.g. a remote dentist 26 in an urban region. The control device 24 is, for example, connected to a display 28 displaying the image 22.

The step of determining the collection site 18 on the tissue of interest 10 by using the provided image 22, and the subsequent step of determining a relative position of the tissue sample 14 in the tissue 10 according to the determined excision site 18 may be performed by the remote dentist 26 or a local person. Therefore, light microscopy and human evaluation by a clinician, dentist or pathologist may be used. Alternatively, one or both steps may be performed by the control device 24 using computer-aided image analysis, e.g. by using a suitable algorithm that e.g. allows determining specific cell structures and which can e.g. assist with the interpretation of e.g. a likelihood of cancer. Such algorithm may e.g. comprise a commonly known algorithm for image recognition. The dentist 26 may use a telecommunication device 32 as known in the art to e.g. provide advice or feedback to patient 12 and/or the rural medical assistant performing e.g. the biopsy.

An image of the tissue sample 14 itself may be provided by e.g. taking a microscopic picture 22' (S3). For example, a microscope 32 can be connected to a smart phone, a tablet computer or a camera and a computer with e.g. internet access allow taking and sending of the image 22' (S4) to the remote control device 24 (S4). In the drawing, microscope image 22' shows several cells 34 with cell nuclei 36. The image 22' can then, for example, be analyzed and stored by analysis software in a cloud or on a remote server as control device 24 and be sent, e.g., to the dentist 26 for diagnosis and/or medical advice. Such a set-up allows e.g. to perform cancer screenings by personnel with limited training in a poorly-equipped environment, for example in rural areas without electricity, and/or limited medical infrastructure. For example, a small, mobile and battery-driven microscope 32 connected to a smart phone or tablet could therefore make the need for a well-equipped laboratory with a conventional microscope 32 and elaborate image analysis software unnecessary.

The procedural steps of transferring the images 22, 22' to control device 24 are merely represented by a schematic in the drawing. The telemicroscopy framework of how e.g. the images 22' is transferred may comprise using e.g. a smartphone-based microscope, i.e. e.g. a device that may be attached and/or connected to a mobile end device, e.g. a smartphone, for capturing the microscopic image 22' with e.g. an application software ("app"). The image transmission may comprise viewing and reviewing of the image 22' on the mobile end device, and/or triaging information from e.g. a remote clinician.

For example, the transfer of image 22' comprises using a cell phone network and/or the internet. The image 22' is then e.g. transmitted to a data store of e.g. a cloud environment. A processing server of the cloud may then perform an image analysis.

The image 22' can additionally or alternatively be transmitted from the cloud environment to a web browser via a web portal, and/or the internet. The image 22' may be viewed and/or reviewed in the web browser and/or via the web portal by the clinician 26, having access to the web browser. Screening results may be sent back to field, i.e. the local technician, for appropriate triaging, via the web-portal, the cloud environment, and/or the internet, preferably using the data transfer as described above.

Alternatively, or additionally, as shown in the drawing, the sensor device 20 or a further sensor device 20 provides e.g. a video image 22 sequence of the tissue of interest 10 (S2). Such video image 22 sequence may be used to supervise the process of e.g. labeling the tissue of interest 10, and/or supervising a biopsy step. In case patient 12, for example, uses a mouth rinse as labeling means 16, the labeled tissue of interest 10 can be shown in such video image sequence even before the biopsy. An image 22 of such video image 22 sequence may then show the unaffected tissue of interest 10, and the dentist 26 may, after the transmission of the image sequence (S5), use such image 22 to determine the collection site 18 for biopsy.

In a further example, the method of the invention may be performed as described above for the drawing. Instead of using a biopsy brush as labeling device 16, the labeling device 16 may comprise a scanning device for a smartphone. Such scanning device may be used to take a panoramic image 22 of the oral captivity, i.e. be used as labeling means 16 and as sensor device 20 at the same time. If e.g. a malignant cancer lesion in the tissue of interest 10 is illuminated by e.g. blue fluorescent light at a particular wavelength that is emitted by a light source of the scanning device, only the malignant cancer lesion, i.e. cells 34 with specific tissue properties, appear e.g. as areas of differential contrast. Such device is described in the prior art and thus known to the skilled person.

As an example, a patient 12 may take a panoramic image 22 as baseline reference of the entire mouth region using such scanning device. Such baseline reference may be used to determine and/or digitally label different subregions of the oral captivity. For example, the macroscopic panorama of a patient's mouth is divided into quadrants (or other scheme for referring to locations), like "upper left", "upper right", "lower left", "lower right" quadrant.

In addition to that, the patient 12 may take additional images 22 of the subregions using the scanning device or a photo camera. In this way, one or more images 22' of the tissue sample 14 and/or one or more images 22 of the subregions can be assigned to a position in the oral captivity.

For example, a biopsy or biopsies may then be performed at different locations, i.e. sites. For example, three of the sites ("#1", "#2", "#3") are captured by the field technician, but a fourth site ("#4") was missed by the field technician, but identified by a remote clinician 26 as being suspicious after seeing the panoramic image 22 shared via telemedicine. Instructions can be sent to the field technician to capture this location also (e.g. also using a telemedicine framework).

In this example, for each of the four sites e.g. a brush biopsy is performed, and cells are transferred onto a glass slide for microscopic analysis. The microscopic images of the biopsied tissue can then be correlated back to where the tissue was obtained in the oral cavity. In this example, the tissue biopsied from locations #1, #3 and #4 were all found to be benign, whereas #2 was found to contain cancerous cells. Based on this, the clinician 26 now has a record of where in the oral cavity the cancerous tissue came from. In this case, it allows the clinician 26 to discriminate between two biopsies that both came e.g. from the upper right quadrant of the oral cavity (one benign and one malignant).

Such process improves the interpretation of tissue samples 14 and improves the reliability of a diagnosis. Such method can also be used as efficient tool for a preventative screening of tissues.

In summary, the examples described above provide a method and/or tool to correlate the gathering of cells 34 by e.g. brush biopsy to e.g. a specific oral region. Therefore, it elaborates a way of identifying the location of where the biopsy, preferably the brush biopsy, was taken in the mouth (correlating to a microscopic image 22' captured with e.g. a microscope device, with the macroscopic anatomy of the disease). Some possible methods for this could be: 1) having a colored mouth "rinse" or rinsing agent that the patient 12 swishes around in his or her mouth before the biopsy and then when the brush is used, this would remove the color, identifying where the brush biopsy was taken, or 2) using a specialized brush biopsy wand 16 that was impregnated with e.g. some kind of color (or fluorescent) marker that remains after the brush was used. The invention could therefore e.g. be a disposable impregnated brush 16 for brush biopsy or a special non-toxic mouth rinsing agent. As an example, the practicing medical worker or field technician could take a picture 22 or a movie 22 of the patient 12 mouth after gathering the samples for brush biopsy e.g. with a smart phone camera 20 that would be sent together with the microscopic images 22' of the brush biopsy (e.g. from a mobile device) using a telemedicine approach. This would allow a remote medical 26 expert to see both the lesion in the patient's 12 mouth and the oral region 10 where the brush biopsy was acquired. Therefore he/she could correlate the macroscopic anatomy with the disease and e.g. give feedback via a telemedicine approach (e.g. by phone, email or other electronic messaging) to the practicing medical worker 26 at the remote location if the right region was chosen for the brush biopsy.

This way, the remote medical expert 26 could not only help with the interpretation of the microscopy images 22', but also assist with the sample 14 acquisition by brush biopsy (i.e. instructing a field worker where to sample or re-sample). For example, a digital photograph 22 of e.g. the mouth cavity after either scenario would have to be used to capture the location of the brush biopsy. Pictures or movies of the oral cavity could also be taken before a brush biopsy is performed and shared with a remote expert 26 who could also use this information to help guide the field worker as to where to perform the technique.

The invention allows the correlation of a brush biopsy to a specific region in the mouth. In combination with the workflow described above, this could allow e.g. a remote medical expert 26 not only to evaluate the microscopic picture 22' but also to correlate them with the macroscopic anatomy, and to control the correct execution of the brush biopsy. This improves the reliability of diagnosis or other patient triaging. The invention helps to improve the acquisition of e.g. brush biopsy samples taken by less experienced personnel (e.g. in a nonclinical environment, or remote location) by providing a telemedicine framework that allows them send images of the region to remote experts for interpretation and support.

The approach may be done remotely (i.e. using telemedicine) or locally (e.g. by a clinician or a dentist in their office or in a surgery room). In both cases, a record of associating the biopsy with where it came from e.g. in the mouth is supported by the method of the invention. A telemedicine approach is preferred, as it allows e.g. the clinician to e.g. direct e.g. a field technician and tell him or her where to take a biopsy, if e.g. a potentially interesting area is missed.

## Claims

1. Method for providing information on a relative position of a tissue sample (14), wherein the tissue sample (14) is a collected part of a tissue of interest (10) of a patient (12), comprising the steps of:
- providing an image (22) of the tissue of interest (10) using a sensor device (20, S2),
- determining a collection site (18) on the tissue of interest (10) by using the provided image (22), and
- according to the determined excision site (18), determining the relative position of the tissue sample (14).

2. Method of claim 1,
**characterized in that**
- the tissue of interest (10) comprises the collection site (18) and determining the collection site (18) comprises determining the site where the tissue sample (14) has been collected , or
- the tissue of interest (10) is unaffected and determining the collection site (18) comprises determining the site where the tissue sample (14) is to be collected.

3. Method of any one of the preceding claims,
**characterized by** the step of:
- labeling the tissue of interest (10) using a labeling means (16) prior to providing the image (22) of the tissue of interest (10, S1).

4. Method of claim 3,
**characterized in that**
- the tissue of interest (10) is labeled by irradiating the tissue of interest (10) with fluorescent light, and/or irradiating or illuminating the tissue of interest with light, and capturing a fluorescence emitted by the tissue using a fluorescence sensor (S1).

5. Method of claim 3 or 4,
**characterized in that**
- the tissue of interest (10) is labeled with a rinsing agent comprising a stain before the tissue sample (14) is collected (S1), or
- the tissue of interest (10) is labeled with a biopsy device as labeling device (16, S1), preferably with a label-impregnated biopsy device (16).

6. Method of any one of the preceding claims,
**characterized in that**
the image (22) is part of an image sequence, preferably avideo image sequence.

7. Method of any one of the preceding claims,
**characterized by**
the following step:
- providing the tissue sample (14) and/or an image (22') of the tissue sample (14, S3, S4), wherein determining the relative position of the tissue sample (14) involves using the provided image (22') of the tissue sample (14) and/or the provided tissue sample (14).

8. Method of claim 7,
**characterized in that**
- providing of the image (22') of the tissue sample (14, S3, S4) comprises capturing the image (22') using a sensor device, in particular a photo camera, frame camera, or video camera, of the microscope (32) or of a smartphone-based microscope.

9. Method of any one of the preceding claims,
**characterized by**
the following step:
- determining a trait of a cell type (34) of the tissue sample (14).

10. Method of any one of the preceding claims,
**characterized in that**
at least one step of providing an image (S2, S4, S5) includes transmitting the image (22, 22', 22") via a wireless or wire-bound communication link to a control device (24), in particular to a remote control device (24).

11. Method of claim 10,
**characterized in that**
the control device (26) performs the step of determining the collection site (18) on the tissue of interest (10) and/or the step of determining the relative position of the tissue sample (14).

12. Method for diagnosing a pathological condition of a tissue sample (14),
**characterized by**
- labeling a tissue of interest (10) with fluorescent light, a stain or a dye using a labeling device (16, S1),
- providing an image (22) of the labeled tissue of interest (10, S2),
- determining a collection site (18) on the labeled tissue of interest (10) by using the provided image (22), and
- determining a pathological condition of the ex situ tissue sample (14) subject to the determined excision site (18).

13. Method according to claim 12,
**characterized by**
the step of obtaining the tissue sample (14) from the tissue of interest (10) of a patient (12) is performed or after labeling the tissue of interest (10).

14. Labeling means (16) for use in a method according to any one of claims 1 to 13,
**characterized by**
comprising
- a stain or dye, and a biopsy brush and a stain or dye for applying the stain on the tissue of interest (10), and/or
- a rinsing agent comprising a stain or dye.
